(19) Europäisches Patentamt
European Patent Office
Office européen des brevets

(11) Publication number: **0 275 431 B1**

(12) # EUROPEAN PATENT SPECIFICATION

(45) Date of publication of patent specification: **23.09.92**

(51) Int. Cl.5: **C07H 15/252**, A61K 31/70

(21) Application number: **87117791.1**

(22) Date of filing: **02.12.87**

The file contains technical information submitted after the application was filed and not included in this specification

(54) **Novel anthracycline derivatives, a process for preparing same and their use as medicaments.**

(30) Priority: **05.12.86 JP 288993/86**

(43) Date of publication of application:
**27.07.88 Bulletin 88/30**

(45) Publication of the grant of the patent:
**23.09.92 Bulletin 92/39**

(84) Designated Contracting States:
**AT BE CH DE ES FR GB GR IT LI LU NL SE**

(56) References cited:
**EP-A- 0 116 222**

**THE JOURNAL OF ANTIBIOTICS, vol. 39, no. 5, May 1986, pages 731-733, Japan Antibiotics Research Association, Tokyo, JP; T. TSUCHIYA et al.: "Syntheses and antitumor activities of 7-0-(2,6-dideoxy-2-fluoro-alpa-L-talopyranosyl)-daunomycinone and -adriamycinone"**

(73) Proprietor: **MICROBIAL CHEMISTRY RESEARCH FOUNDATION**
**14-23, Kamiosaki 3 Chome Shinagawa-ku**
**Tokyo 141(JP)**

(72) Inventor: **Umezawa, Hamao**
**deceased(JP)**
Inventor: **Umezawa, Sumio**
**5-1-1-709, Shinjuku Shinjuku-ku**
**Tokyo(JP)**
Inventor: **Tsuchiya, Tsutomu**
**3-1-17-201, Eda-higashi Midori-ku**
**Yokohama City Kanagawa Prefecture(JP)**
Inventor: **Takeuchi, Tomio No. 701A, New Fuji Mansion 5-1-11**
**Higashi-gotanda 5-chome,**
**Shinagawa-ku Tokyo(JP)**
Inventor: **Takagi, Yasushi**
**98-5, Higashi-kibougaoka Asahi-ku**
**Yokohama City Kanagawa Prefecture(JP)**
Inventor: **Yoneda, Toshio 13-908, Minami-kandaiji**
**Kandaiji-cho Kanagawa-ku**
**Yokohama City Kanagawa Prefecture(JP)**
Inventor: **Fukatsu, Shunzo**
**35-2, Haraikata-cho**
**Shinjuku-ku Tokyo(JP)**

(74) Representative: **Vossius & Partner**
**Siebertstrasse 4 P.O. Box 86 07 67**
**W-8000 München 86(DE)**

EP 0 275 431 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel anthracycline derivatives and a process for preparing the same.

The anthracycline antibiotics thus far known are daunomycin (see U.S. Patent 3,616,242) and adriamycin (see U.S. Patent 3,590,028) which are obtained from the cultured broths of actinomycetes. These compounds have broad anticancer or carcinostatic spectra against experimental tumors, and are widely used clinically as cancer chemotherapeutics.

Daunomycin and adriamycin are compounds expressed by the following general formula (a)

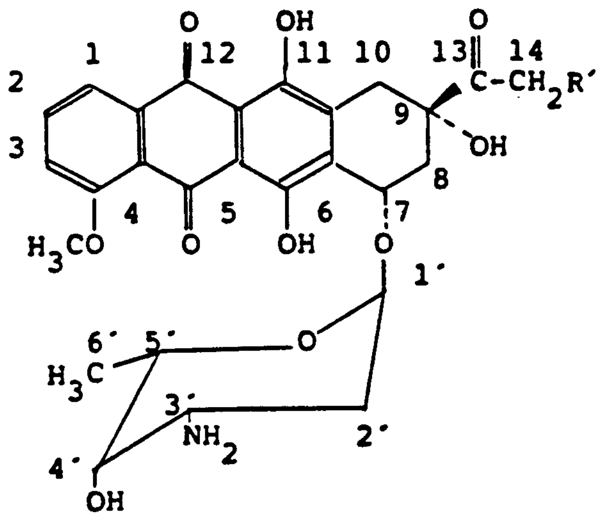

(a)

wherein R′ represents a hydrogen atom or a hydroxyl group.

We, the inventors of this invention, have proposed novel compounds of the following formula (b) that have higher antitumor activity than that of daunomycin and adriamycin (Japanese Patent Application No. 282798/85 corresponding to EP-A-230 013).

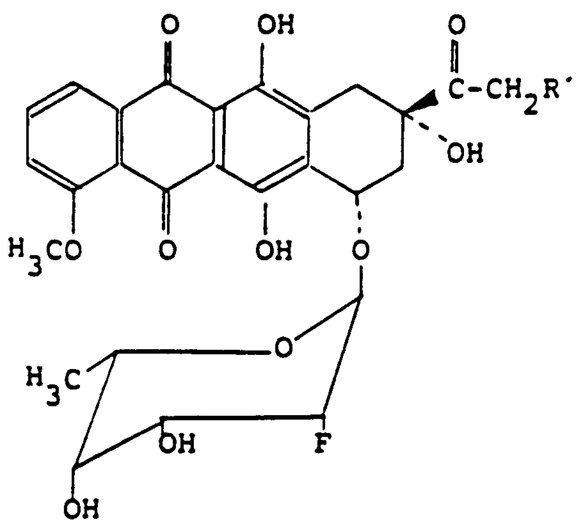

(b)

wherein R' represents a hydrogen atom or a hydroxyl group.

Anthracycline derivatives of the formula (b), however, have poor solubility in water, and there have been various restrictions on their use as injections. We have conducted varieties of studies in attempts to improve the water solubility and other properties of the compounds of the formula (b) to apply them as injections or capsules.

The Journal of the Antibiotics, Vol. XXXIX, no.5, p. 731-733 discloses 7-0-(2,6-dideoxy-2-fluoro-α-L-talopyranosyl)daunomycinone and -adriamycinon and their antitumor activity. EP-A 116 222 discloses 14-Acyloxy-2'-halo-anthracyline antibiotics and their use as anti-cancer agent.

As described earlier, the antitumor activity of novel compounds expressed by the general formula (b) is by far better than that of daunomycin and adriamycin (Japanese Patent Application No. 282798/85). We, therefore, made various studies with a view to create derivatives, from the compounds of the formula (b), while retaining the high antitumor activity of the compounds of the formula (b) and having improved solubility in water. These studies have resulted successfully in synthesizing novel derivatives esterified at the 14-position as shown in the general formula (I) below, and such novel derivatives have been found to attain the desired objectives of this invention.

In more detail, a first aspect of this invention provides anthracycline derivatives of the general formula (I)

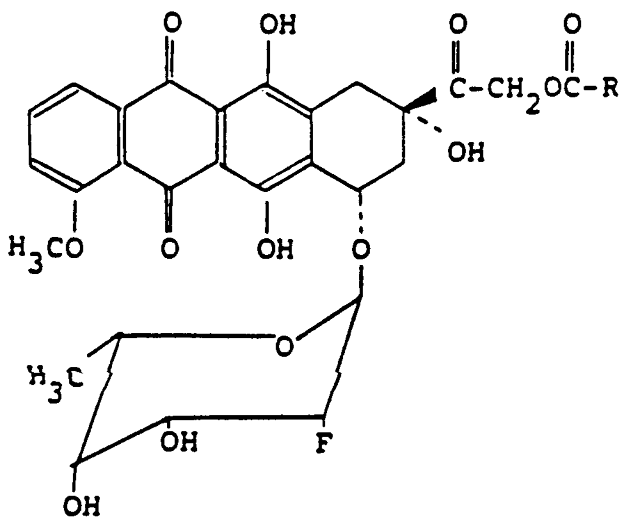

(I)

wherein R represents the group $-(CH_2)_mH$ in which m denotes 0 or an integer of 1 to 6, or the group $-(CH_2)_nCOOH$ in which n denotes 0 or an integer of 1 to 10.

Preferred are the compounds wherein R is the group $-(CH_2)_nCOOH$ and n denotes an integer of 2 to 6.

Examples of the preferred compounds of the general formula (I) include 6 compounds as shown in Examples 1(b) through 6(b) to be given later, including 7-0-(2,6-dideoxy-2-fluoro-α-L-talopyranosyl)-adriamycinone 14-0-hemipimelate.

These compounds exemplified in Examples 1(b) through 6(b) were tested for solubility in water and antitumor activity. The results are shown below.

(1) Solubility

The respective examples of the compounds of the formula (I) according to this invention showed the following solubilities (measured at room temperature) in phosphate buffer solution (pH: 7.4). The phosphate buffer solution was prepared by mixing 0.05M potassium dihydrogenphosphate ($KH_2PO_4$) with an aqueous solution of sodium hydroxide.

Table 1

| Compounds of this invention | Solubility (mg/ml) |
|---|---|
| Compound 3 of Example 1(b) | < 1 |
| Compound 5 of Example 2(b) | 25 |
| Compound 7 of Example 3(b) | 26 |
| Compound 9 of Example 4(b) | 33 |
| Compound 11 of Example 5(b) | 33 |
| Compound 13 of Example 6(b) | 30 |
| Control | < 1 |

Control: 7-O-(2,6-dideoxy-2-fluoro-α-L-talopyranosyl)adriamycinone

(2) Antitumor activity

To assess the antitumor activity of the compounds of this invention against tumors in test animals, 1 x $10^5$ mouse leukemia L-1210 cells/mouse were transplanted intraperitoneally to $CDF_1$ mice. The compounds according to this invention was administered intraperitoneally for 9 consecutive days, beginning 24 hours after transplantation, and the animals were observed for 30 days. The survival time, in days, of the compound-treated group (T) was calculated, and that of the control group (C) receiving physiological saline solution alone was also calculated. Then, the increase of life span (T/C, %) was determined. The results are shown in Table 2.

Table 2

| Compound of this invention | Increase of life span (T/C, %) | | | | | |
|---|---|---|---|---|---|---|
| | Dose (mg/kg/day) | | | | | |
| | 5 | 2.5 | 1.25 | 0.6 | 0.3 | 0.15 |
| Compound of Example 1(b) | >306* | >282 | >353 | >353 | 135 | 135 |
| Compound of Example 2(b) | >353* | >353 | >292 | >124 | 124 | |
| Compound of Example 3(b) | >306* | >294 | 282 | >235 | 129 | 129 |
| Compound of Example 4(b) | >353 | >353 | 265 | 165 | 141 | 112 |
| Compound of Example 5(b) | >353 | >271 | >294 | 176 | 171 | 165 |

* Toxic reaction occurred.

As demonstrated in Tables 1 and 2, some of the compounds of formula (I) in accordance with this invention

remarkably improved solubility in water, while retaining high antitumor activity. Accordingly, their use as injections has become much easier.

A second aspect of this invention provides a process for preparing an anthracycline derivative of the formula (I)

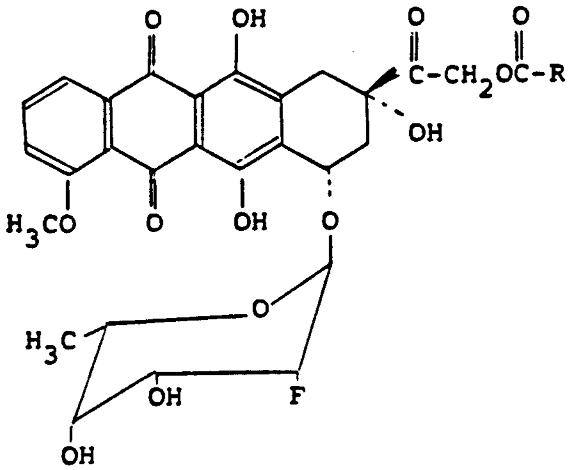

(I)

wherein R represents the group $-(CH_2)_mH$ in which m denotes 0 or an integer of 1 to 6, or the group $-(CH_2)_nCOOH$ in which n denotes 0 or an integer of 1 to 10, which comprises reacting a compound of the general formula (II)

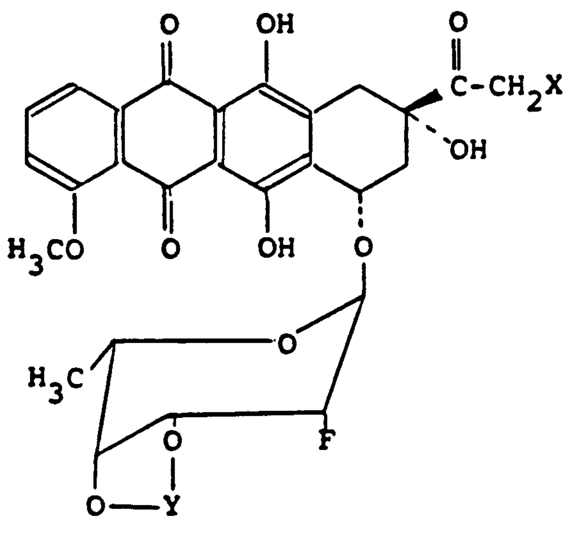

(II)

wherein X represents a bromine, chlorine or iodine atom, and Y represents a divalent hydroxyl-protecting group, or the corresponding compound from which the hydroxyl-protecting group Y is removed, with a carboxylic acid alkali metal salt of the formula (III)

A-OOC-R (III)

wherein R is as defined above, and A represents an alkali metal, to form a compound of the formula (IV)

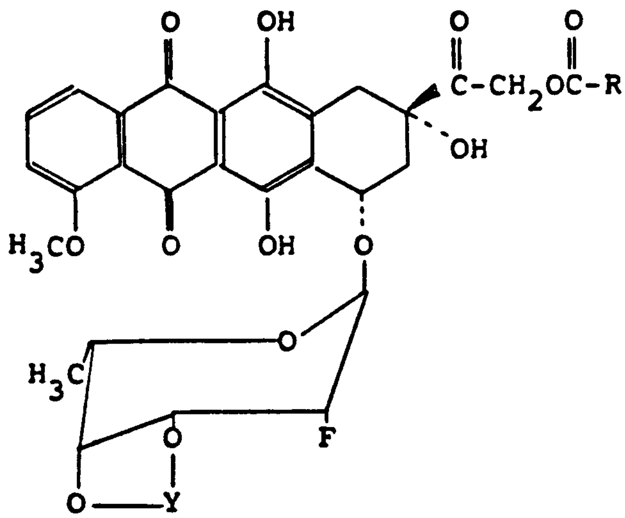

(IV)

wherein R and Y are as defined above, or the corresponding compound from which the hydroxyl-protecting group Y is removed; and then, if the resulting compound contains the hydroxyl-protecting group Y remaining therein, removing the hydroxyl-protecting group Y from said compound by a customary method.

In the case of the compound of formula (II) in which X is a bromine atom and Y is an isopropylidene group, the process for preparing the compound of the general formula (I) proceeds according to the following reaction scheme:

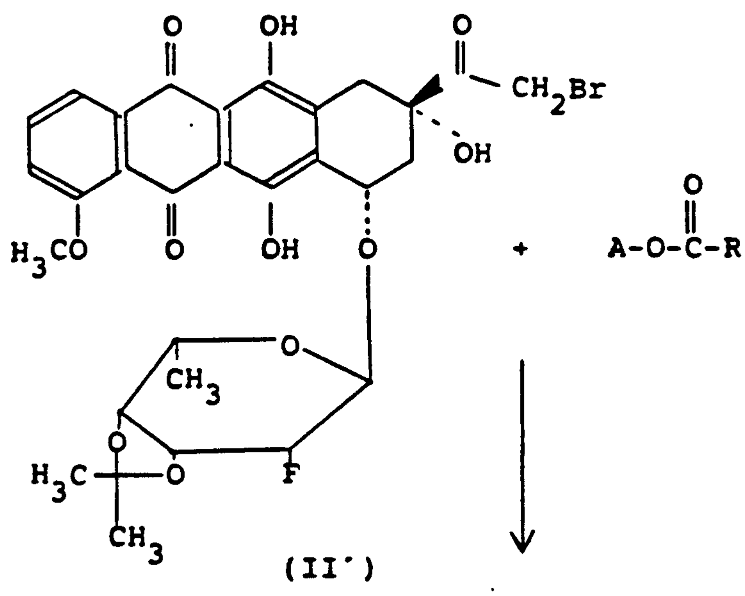

(III)

(II')

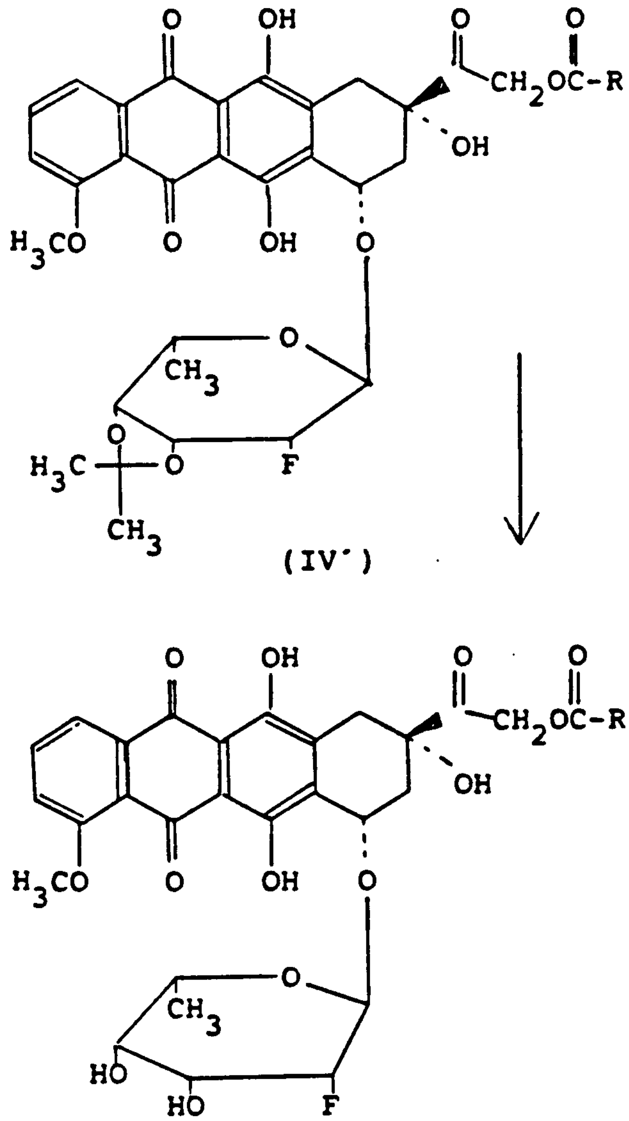

(IV')

(I)

In the above reaction scheme, R represents the group $-(CH_2)_mH$ in which m denotes 0 or an integer of 1 to 6, or the group $-(CH_2)_nCOOH$ in which n denotes 0 or an integer of 1 to 10, and A represents an alkali metal, such as sodium, potassium or lithium.

The compound of formula (II) having an isopropylidene group as Y, the starting material in the process of this invention, can be prepared in the following manner: A protected derivative of the compound of the aforementioned formula (b), wherein R′ = H, shown in the specification of Japanese Patent Application No. 282798/85, said derivative in which the carbonyl group at the 13-position of 7-0-(2,6-dideoxy-2-fluoro-α-L-talopyranosyl)daunomycinone has been protected with a ketal group, is brominated with bromine, chlorinated with chlorine (preferably under irradiation with light), or iodinated with iodine at the 14-position of said derivatives by the method described in Japanese Laid-Open Patent Publication No. 156300/81. The resulting halogenation product is deprived of the ketal group at the 13-position in acetone as a solvent. Simultaneously with this reaction, there occurs a side reaction in which the hydroxyl groups at the 3- and 4-positions of the talose moiety of the halogenation product are converted into a form protected with an isopropylidene group upon reaction with acetone.

To prepare the compound of formula (IV) by condensation from the compound of formula (II) and the compound of formula (III) in the process of this invention, the reaction is performed at a temperature of 0 to 100 °C., usually in the vicinity of room temperature, for 5 to 30 hours, usually 15 hours. The solvent for use in the reaction is acetone, tetrahydrofuran, methanol, ethanol, DMF, DMSO, or a mixture of any of these with water.

When a dibasic acid mono salt, R = $-(CH_2)_nCOOH$, is used as the carboxylic acid salt of formula (III), it is important that exactly one of the two carboxylic acids is reacted with the compound of formula (II).

In the case of the starting compound of formula (II) for use in the process of this invention, in which the hydroxyl groups at the 3- and 4-positions of the talose moiety are blocked with the protective group Y, the resulting condensation product of formula (IV) contains the hydroxyl-protecting group Y remaining therein. In this case, the hydroxyl-protecting group Y is removed by a customary method to form the desired compound of formula (I). The hydroxyl-protecting group Y in the starting compound of formula (II) may be a known one, preferably, a $C_{2-6}$ alkylidene group, such as a ethylidene or isopropylidene group; an aralkylidene group, such as a benzylidene group; a cycloalkylidene group, such as a cyclohexylidene group; or a tetrahydropyranylidene group. These hydroxyl-protecting groups can be removed by hydrolysis with acids. Acids that can be used for this purpose are lower fatty acids, such as formic acid, acetic acid and trichloroacetic acid, or inorganic acids, such as hydrochloric acid, sulfuric acid and phosphoric acid, and usually, acetic acid is used. Solvents for use in this protective group-removing reaction may be water, alcohol, or mixtures of aprotic solvents, such as DMF, DMSO, dioxane, and tetrahydrofuran, with water or alcohol. Usually, water is used. The reaction temperature is 0 to 100° C., usually 50 to 70° C.

The compound of formula (II) used as the starting compound in the process of this invention is also a novel compound. The compound of formula (II) in which X is a bromine atom and Y is an isopropylidene group is 14-bromo-7-0-(2,6-dideoxy-2-fluoro-3,4-0-isopropylidene-α-L-talopyranosyl)daunomyucine (hereinafter sometimes referred to as compound 1). It is obtained as a red, solid intermediate in Example 2 in the specification of Japanese Patent Application No. 282798/85. The preparation examples for compound 1 will be given later in Referential Examples 1 to 4. The preparation of compound 1 is performed, if described briefly, in the following manner: 3,4-Di-O-acetyl-2,6-dideoxy-2-fluoro-α-L-talopyranosyl bromide (also a novel compound) is reacted with daunomycinone in dichloromethane in the presence of mercuric oxide and mercuric bromide to form 7-0-(3,4-di-O-acetyl-2,6-dideoxy-2-fluoro-α-L-talopyranosyl)-daunomycinone. The resulting compound is hydrolized in an aqueous solution of sodium hydroxide for deacetylation, thereby forming 7-O-(2,6-dideoxy-2-fluoro-α-L-talopyranosyl)daunomycinone. This compound is reacted with methyl orthoformate in a mixture of anhydrous methanol and dixoane, to protect the carbonyl group at the 13-position of said compound by converting it into methylketal (see Japanese Patent Publication No. 36919/82). The resulting protected derivative with ketal is reacted with bromine to brominate the methyl group at the 14-position. The resulting 14-brominated derivative is suspended in acetone for reaction with it, thereby removing that group which has protected the 13-position carbonyl group in the form of methylketal, to restore the carbonyl group. Simultaneously, there occurs a side reaction in which acetone is reacted incidentally with the hydroxyl groups at the 3- and 4-positions of the talose moiety, whereby an isopropylidene group is introduced between the hydroxyl groups at the 3- and 4-positions (see Referential Examples 1 to 4 to be given later).

14-Bromo-7-0-(2,6-dideoxy-2-fluoro-3,4-0-isopropylidene-α-L-talopyranosyl)daunomycinone (compound 1), if hydrolyzed with a weak acid, has its 3,4-0-isopropylidene group removed, to form 14-bromo-7-0-(2,6-dideoxy-2-fluoro-α-L-talopyranosyl)daunomycinone. This compound is also usable as the starting compound in the process of this invention. If it is reacted with a carboxylic acid alkali metal salt of the formula (III) in accordance with the aforementioned procedure, the desired compound of the formula (I) is formed.

Assume that 14-bromo-7-0-(2,6-dideoxy-2-fluoro-α-L-talopyranosyl)daunomycinone is reacted with a hydroxyl-protecting reagent, such as acetone, 2,2-dimethoxypropane, benzaldehyde, cyclohexanone or 4,4-dimethoxy tetrahydropyran, intended for introduction of an alkylidene, aralkylidene, cycloalkylidene or tetrahydropyranylidene group - known as a divalent hydroxyl-protecting group - by a known hydroxyl-protecting technique. This reaction results in the protection of the 3- and 4-hydroxyl groups of the talose moiety with the aforesaid divalent hydroxyl-protecting group Y, to give the compound of the general formula (II). Once the aforementioned 14-bromo- or 14-halo-7-0-(2,6-dideoxy-2-fluoro-α-L-talopyranosyl) daunomycinone is obtained, however, it can directly be used for reaction with the carboxylic acid alkali metal salt of formula (III), without the protection of the hydroxyl groups of its talose moiety.

The compounds of the present invention can be used as the active ingredient in pharmaceutical compositions in association with a pharmaceutically acceptable carrier.

This invention will be described in more detail with reference to Examples 1 to 6, and Referential Examples 1 to 4 that show the preparation of the starting compound.

## Example 1

(a) Preparation of 7-0-(2,6-dideoxy-2-fluoro-3,4-0-isopropylidene-α-L-talopyranosyl)adriamycinone 14-0-valerate (Compound 2)

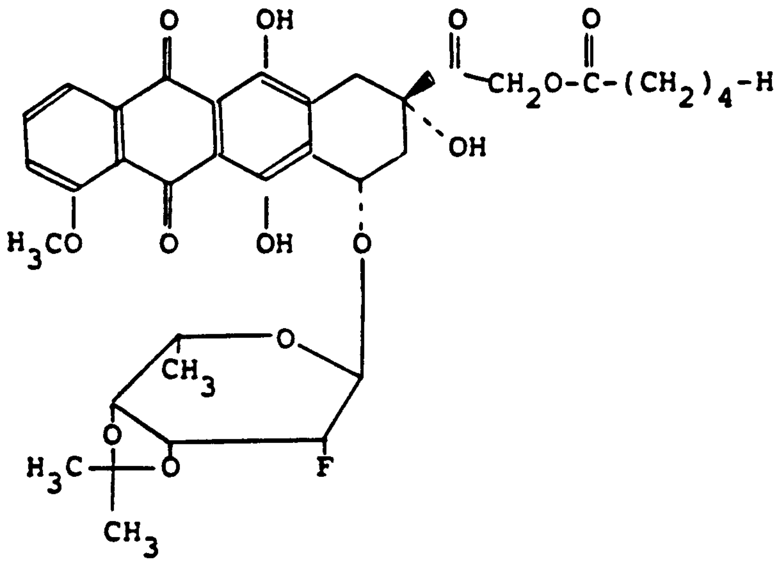

(Compound 2)

206 Milligrams of 14-bromo-7-0-(2,6-dideoxy-2-fluoro-3,4-0-isopropylidene-α-L-talopyranosyl)-daunomycinone (Compound 1) was dissolved in a mixture of 20 ml of acetone and 5 ml of water. 690 Milligrams of sodium valerate was added to the solution, and the mixture was stirred for 14 hours at room temperature. Acetone was distilled off under reduced pressure, and the residue was extracted with chloroform. The chloroform layer was washed with water, dried over anhydrous sodium sulfate, and solidified to dryness under reduced pressure. The residue was purified by silica gel column chromatography (chloroform-methanol = 50:1 by volume) to obtain 102 mg (48.1%) of the captioned compound as a red solid.

mp. 172 - 178° C
$[\alpha]_D^{25}$ + 60° (c 0.079, $CHCl_3$)
MS (FD) : 686 ($M^+$)

(b) Preparation of 7-0-(2,6-dideoxy-2-fluoro-α-L-talopyranosyl) adriamycinone 14-0-valerate (Compound 3)

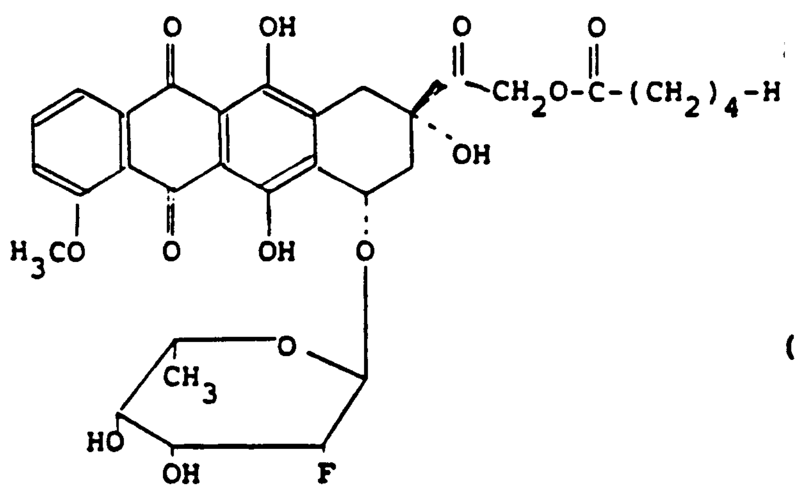

(Compound 3)

The 7-0-(2,6-dideoxy-2-fluoro-3,4-0-isopropylidene-α-L-talopyranosyl)adriamycinone 14-0-valerate (Compound 2, 61 mg) obtained in the step (a) was dissolved in 6 ml of an aqueous solution of 80 % acetic acid, and the solution was held at 70° C for 40 minutes. The solvent was distilled off under reduced pressure, and the residue was purified by preparative silica gel thin-layer chromatography (chloroform-methanol = 20:1) to obtain 43 mg (89%) of the captioned compound as a red solid.

mp. 163 - 169 ° C
$[\alpha]_D^{25}$ + 172 ° (c 0.12, $CHCl_3$-MeOH,1 : 1 by volume)
MS (FD) : 646 ($M^+$)

Example 2

(a) Preparation of 7-0-(2,6-dideoxy-2-fluoro-3,4-0-isopropylidene-α-L-talopyranosyl)adriamycinone 14-0-hemisuccinate (Compound 4)

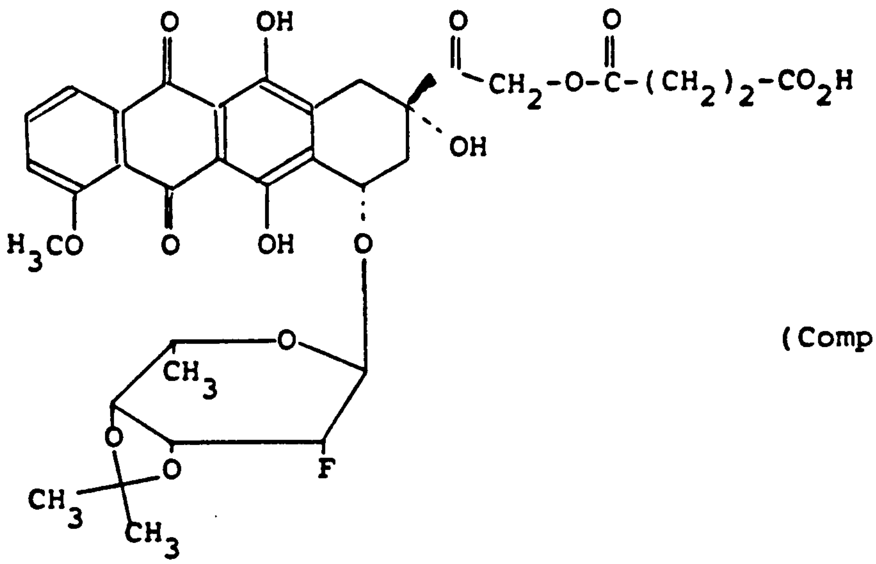

(Compound 4)

100 mg of Compound 1 used in Example 1(a) and 380 mg of monosodium succinate were used as the starting materials, and the same procedure as in Example 1(a) was performed, to obtain 76 mg (72%) of the captioned compound as a red solid. The eluent used for silica gel column chromatography was chloroform-methanol (50:1).
mp. 128 - 132 ° C (decomposition)
$[\alpha]_D^{25}$ + 57 ° (c 0.070, $CHCl_3$)
MS (FD) : 703 (M + 1)

(b) Preparation of 7-0-(2,6-dideoxy-2-fluoro-α-L-talopyranosyl) adriamycinone 14-0-hemisuccinate (Compound 5)

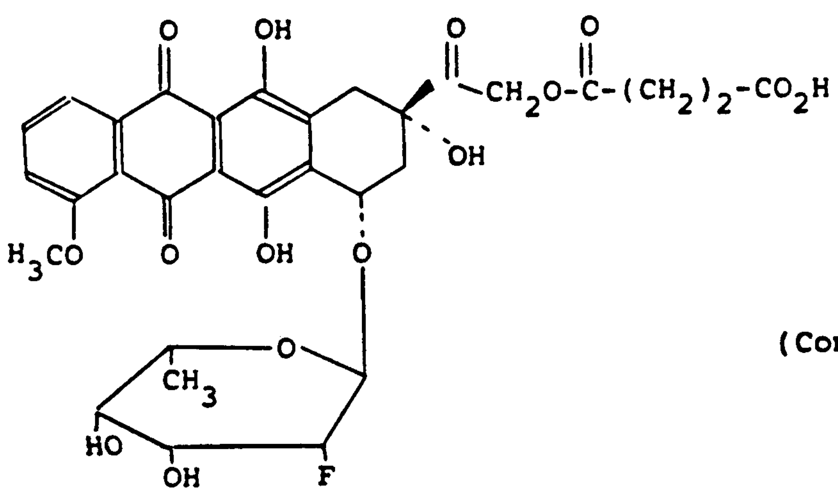

(Compound 5)

56.0 Milligrams of Compound 4 obtained in the step (a) was used as the starting material, and the same procedure as in Example 1(b) was performed to obtain 35 mg (71%) of the captioned compound as a red solid. The developer used for preparative thin-layer chromatography was chloroform-methanol (5:1).
mp. 142 - 146 ° C (decomposition)
$[\alpha]_D^{25}$ + 121 ° (c 0.062, $CHCl_3$-MeOH = 1:1 by volume)
MS (FD) : 662 ($M^+$)

Example 3

(a) Preparation of 7-0-(2,6-dideoxy-2-fluoro-3,4-0-isopropylidene-α-L-talopyranosyl)adriamycinone 14-0-hemiglutarate (Compound 6)

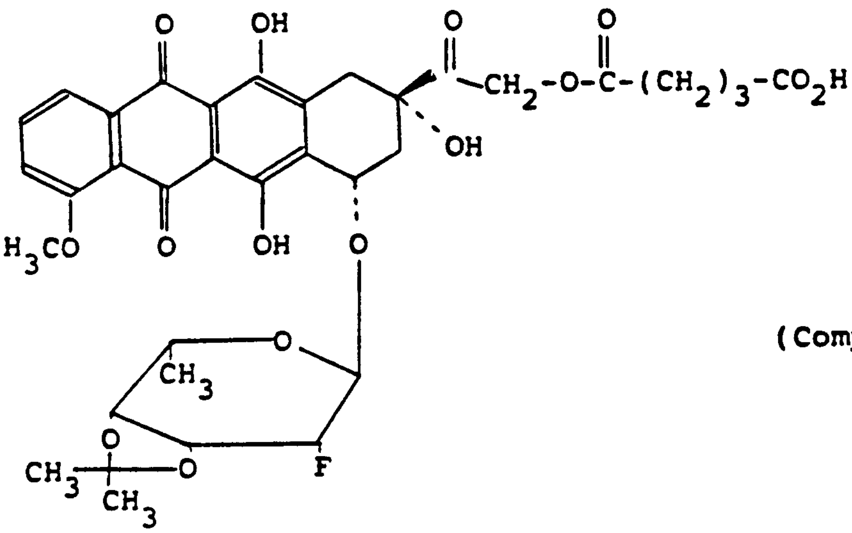

(Compound 6)

100 mg of Compound 1 used in Example 1(a) and 420 mg of monosodium glutarate were used as the starting materials, and the same procedure as in Example 1(a) was performed to obtain 64 mg (59.6%) of the captioned compound as a red solid. The eluent used for silica gel column chromatography was benzene-acetone (4:1).
mp. 135 - 140 ° C
$[\alpha]_D^{25}$ + 65 ° (c 0.12, $CHCl_3$)
MS (FD) : 717 (M + 1)

(b) Preparation of 7-0-(2,6-dideoxy-2-fluoro-α-L-talopyranosyl) adriamycinone 14-0-hemiglutarate (Compound 7)

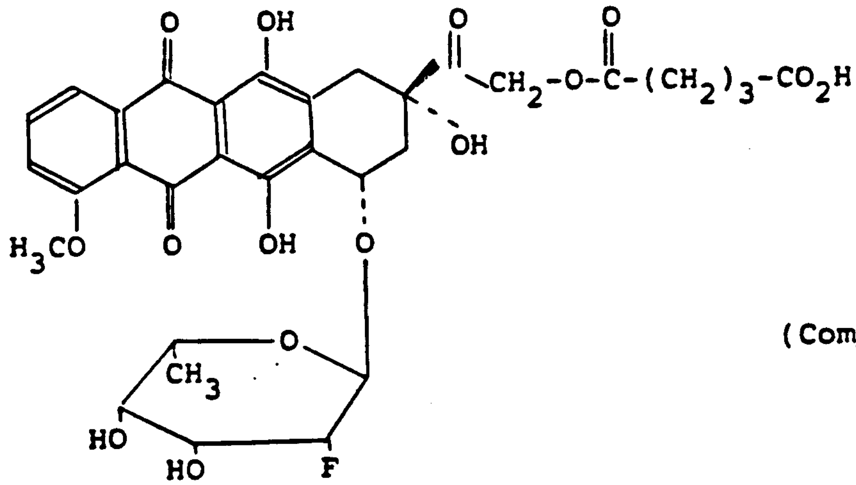

(Compound 7)

73.0 Milligrams of Compound 6 obtained in the step (a) was used as the starting material, and the same procedure as in Example 1(b) was performed to obtain 52.9 mg (76.8%) of the captioned compound as a red solid. The developer used for preparative thin-layer chromatography was chloroform-methanol (5:1).

mp. 163 - 169 °C

$[\alpha]_D^{25}$ + 139° (c 0.035, $CHCl_3$-MeOH, 1:1)

MS (FD) : 676 ($M^+$)

Example 4

(a) Preparation of 7-0-(2,6-dideoxy-2-fluoro-3,4-0-isopropylidene-α-L-talopyranosyl)adriamycinone 14-0-hemiadipate (Compound 8)

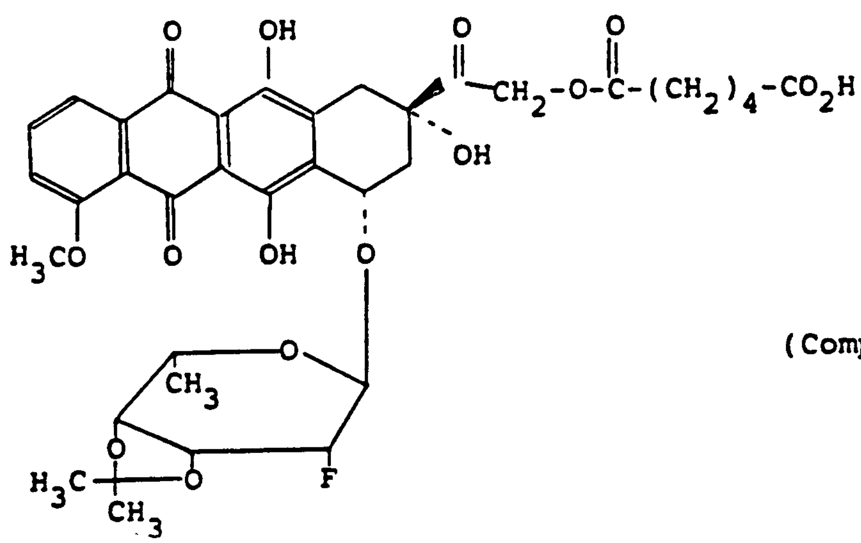

(Compound 8)

123 Milligrams of Compound 1 used in Example 1(a) and 560 mg of monosodium adipate were used as the starting materials, and the same procedure as in Example 1(a) was performed to obtain 89.7 mg (66.5%) of the captioned compound as a red solid. The eluent used for silica gel column chromatography was chloroform-methanol (20:1).

mp. 140 - 145 °C

$[\alpha]_D^{25}$ + 62° (c 0.061, $CHCl_3$)

MS (FD) : 730 ($M^+$)

(b) Preparation of 7-0-(2,6-dideoxy-2-fluoro-α-L-talopyranosyl) adriamycinone 14-0-hemiadipate (Compound 9)

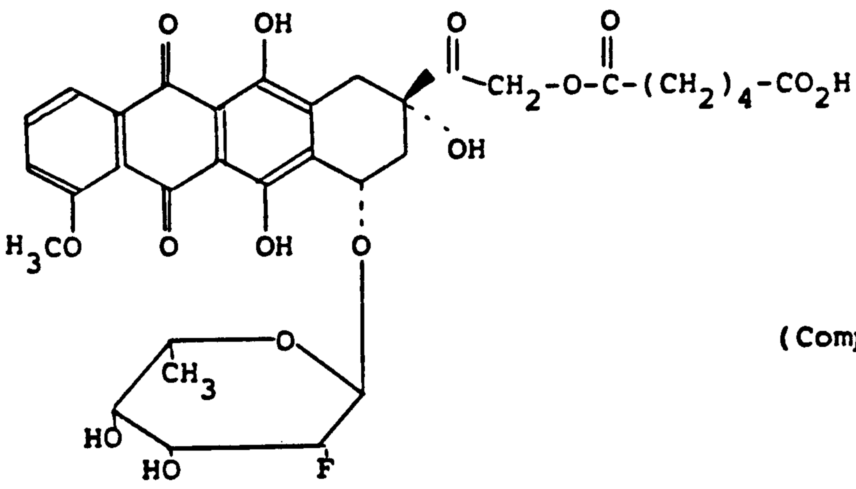

(Compound 9)

58 Milligrams of Compound 8 obtained in the step (a) was used as the starting material, and the same procedure as in Example 1(b) was performed to obtain 44 mg (81%) of the captioned compound as a red solid. The developer used for preparative thin-layer chromatography was chloroform-methanol (10:1).

mp. 135 - 141 °C

$[\alpha]_D^{25}$ + 135° (c 0.069, $CHCl_3$-MeOH, 1:1)

MS (FD) : 691 (M+1)

Example 5

(a) Preparation of 7-0-(2,6-dideoxy-2-fluoro-3,4-0-isopropylidene-α-L-talopyranosyl)adriamycinone 14-0-hemipimelate (Compound 10)

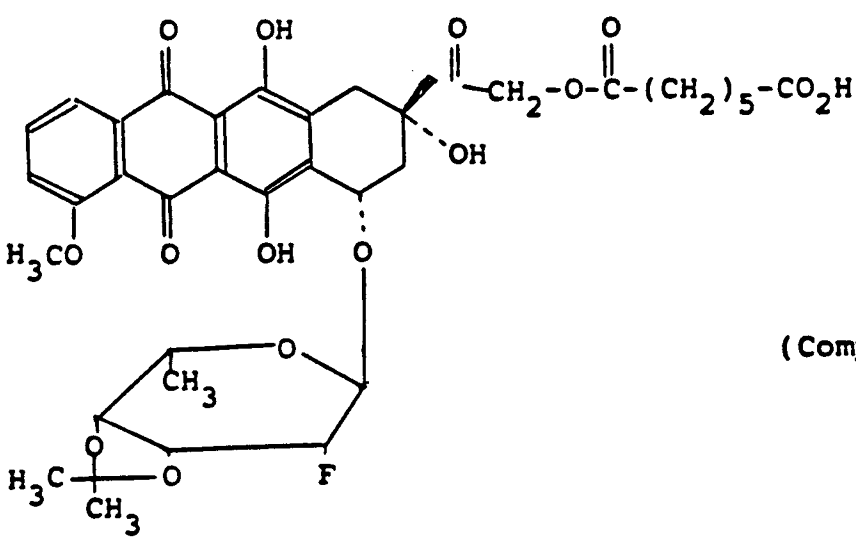

(Compound 10)

100 Milligrams of Compound 1 used in Example 1(a) and 490 mg of monosodium pimelate were used as the starting materials, and the same procedure as in Example 1(a) was performed to obtain 72 mg (64.5%) of the captioned compound as a red solid. The eluent used for silica gel column chromatography was benzene-acetone (5:1).

mp. 131 - 137 °C
$[\alpha]_D^{25}$ + 64 ° (c 0.13, $CHCl_3$)
MS (FD) : 745 (M + 1)

(b) Preparation of 7-0-(2,6-dideoxy-2-fluoro-α-L-talopyranosyl) adriamycinone 14-0-hemipimelate (Compound 11)

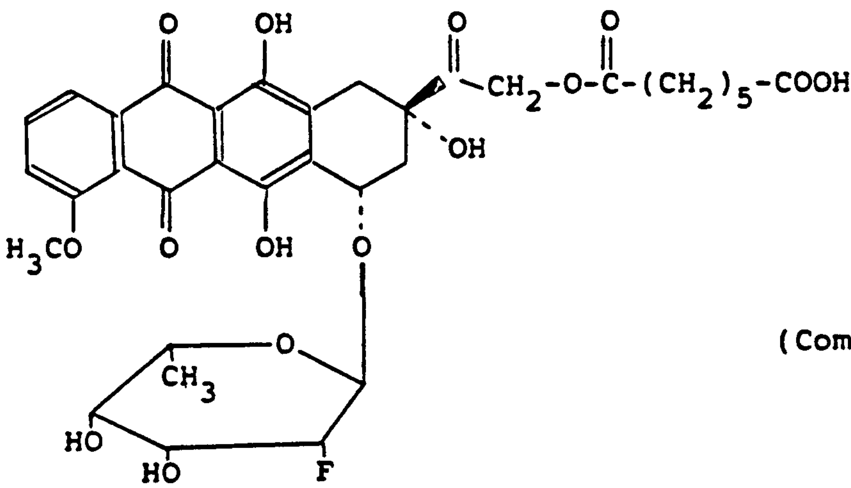

(Compound 11)

75.6 Milligrams of Compound 10 obtained in the step (a) was used as the starting material, and the same procedure as in Example 1(b) was performed to obtain 58.0 mg (80.8%) of the captioned compound as a red solid. The developer for preparative thin-layer chromatography was chloroform-methanol (10:1).
mp. 108 - 113 °C
$[\alpha]_D^{25}$ + 161 ° (c 0.056, $CHCl_3$-MeOH, 1:1)
MS (FD) : 704 ($M^+$)

Example 6

(a) Preparation of 7-0-(2,6-dideoxy-2-fluoro-3,4-0-isopropylidene-α-L-talopyranosyl)adriamycinone 14-0-hemisuberate (Compound 12)

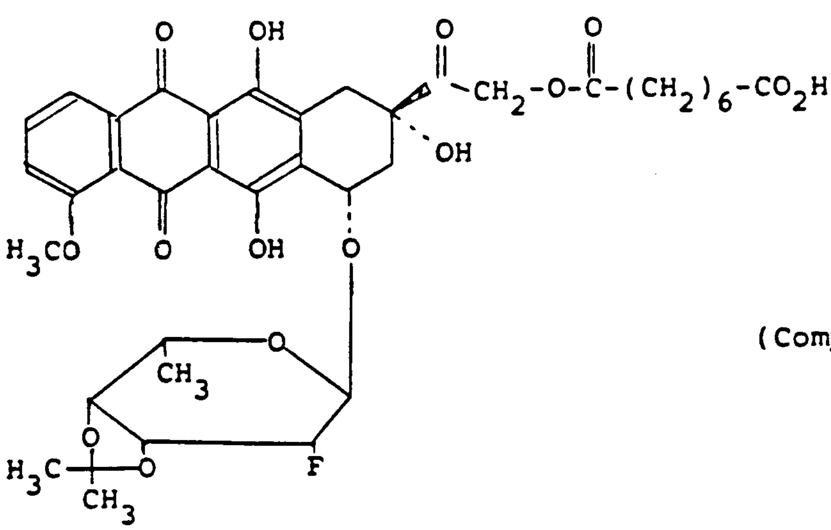

(Compound 12)

106 Milligrams of Compound 1 used in Example 1(a) and 560 mg of monosodium suberate were used as the starting materials, and the same procedure as in Example 1(a) was performed to obtain 81.3 mg (67.3%) of the captioned compound as a red solid. The eluent for silica gel column chromatography was benzene-acetone (6:1).
mp. 132 - 136 °C
$[\alpha]_D^{25}$ + 60 ° (c 0.078, $CHCl_3$)
MS (FD) : 759 (M + 1)

(b) Preparation of 7-0-(2,6-dideoxy-2-fluoro-α-L-talopyranosyl) adriamycinone 14-0-hemisuberate (Compound 13)

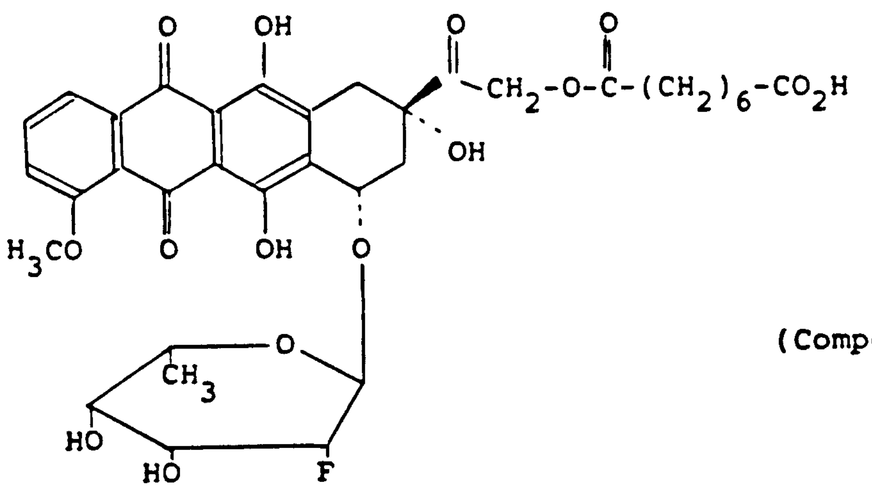

(Compound 13)

85.2 Milligrams of Compound 12 obtained in the step (a) was used as the starting material, and the same procedure as in Example 1(b) was performed to obtain 66.7 mg (82.7%) of the captioned compound as a red solid. The developer for preparative thin-layer chromatography was toluene-ethanol (5:1).
mp. 175 - 178 °C
$[\alpha]_D^{25}$ + 121 ° (c 0.119, $CHCl_3$-MeOH = 1.1 by volume)
MS (FD) : 718 ($M^+$)

Referential Example 1

(1) Preparation of methyl 6-deoxy-3,4-0-isopropylidene-α-L-galactopyranoside

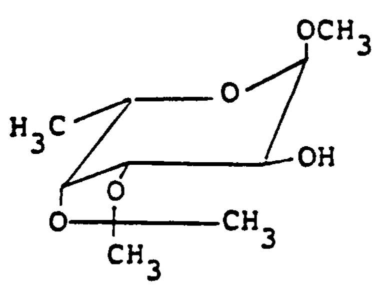

2.90 Grams of L-fucose was suspended in 40 ml of 1% hydrogen chloride-methanol, and the suspension was heated under reflux for 8 hours. The resulting homogeneous solution was cooled to room temperature, and then neutralized with the addition of basic lead carbonate. The mixture was filtered, and the filtrate was concentrated to obtain 3.04 g of a colorless solid composed of a methylfucoside mixture. This solid was dissolved in 40 ml of anhydrous dimethylformamide, and 7.81 g of 2,2-dimethoxypropane and 870 mg of anhydrous p-toluenesulfonic acid were added, followed by reacting the mixture for 2 hours at

room temperature. The reaction mixture was neutralized with the addition of sodium hydrogencarbonate, and the insolubles were removed by filtration. The filtrate was concentrated under reduced pressure, and the residue was dissolved in 100 ml of chloroform. The resulting solution was washed with a saturated aqueous solution of sodium hydrogencarbonate and a 10% aqueous solution of sodium chloride, and then concentrated. The residue was subjected to 400 ml silica gel column chromatography (developer system: hexane-acetone = 3:1) for separation and purification, thereby obtaining 2.28 g (59%) of the captioned compound as a syrup.
$[\alpha]_D^{26}$ - 154° (c 1, chloroform)

(2) Preparation of methyl 2-0-acetyl-6-deoxy-3,4-0-isopropylidene-α-L-galactopyranoside

12.56 Grams of methyl 6-deoxy-3,4-0-isopropylidene-α-L-galactopyranoside was dissolved in 35 ml of anhydrous pyridine, and 17 ml of acetic anhydride was added, followed by reacting the mixture for 8 hours at room temperature. To the reaction mixture was added 20 ml of water, and the resulting mixture was concentrated under reduced pressure. The residue was dissolved in 500 ml of chloroform, and the resulting solution was washed successively with a 10% aqueous solution of potassium hydrogensulfate, saturated aqueous solution of sodium hydrogencarbonate, and water in this order. Then, the solution was concentrated to obtain 13.81 g (92%) of the captioned compound as colorless crystals. Recrystallization from ether-hexane gave needle crystals.
mp. 101 - 102°C
$[\alpha]_D^{26}$ - 176° (c 1, chloroform)

(3) Preparation of methyl 2-0-acetyl-6-deoxy-α-L-galactopyranoside

13.81 Grams of methyl 2-0-acetyl-6-deoxy-3,4-0-isopropylidene-α-L-galactopyranoside was dissolved in 140 ml of an aqueous solution of 80% acetic acid, and the solution was reacted for 1 hour at 80°C. The reaction mixture was concentrated under reduced pressure, and the resulting residue was purified by 600 ml silica gel column chromatography (developer system; hexane-acetone = 1:2) to obtain 11.17 g (96%) of the captioned compound as colorless crystals. Recrystallization was performed from ether-hexane.
mp. 77 - 78°C
$[\alpha]_D^{26}$ - 182° (c 2, chloroform)

(4) Preparation of methyl 2-0-acetyl-6-deoxy-3-0-tosyl-α-L-galactopyranoside

11 Grams of methyl 2-0-acetyl-6-deoxy-α-L-galactopyranosidewas dissolved in 200 ml of anhydrous pyridine, the solution was cooled to -20°C, and 13.33 g of p-toluenesulfonyl chloride was added. The mixture was reacted for 26 hours at that temperature, and for another 19 hours at room temperature. After addition of water, the reaction mixture was concentrated under reduced pressure. The residue was subjected to 700 ml silica gel column chromatography (developer system: hexane-acetone = 1:1) for separation and purification to give 16.25 g (87%) of the captioned compound as colorless crystals. Recrystallization was performed from ether-hexane.
mp. 118 - 120°C
$[\alpha]_D^{26}$ - 136° (c 1, chloroform)

(5) Preparation of methyl 2-0-acetyl-4-0-benzyl-6-deoxy-3-0-toxyl-α-L-galactopyranoside

160 Milligrams of methyl 2-0-acetyl-6-deoxy-3-0-tosyl-α-L-galactopyranoside was dissolved in 3.2 ml of a mixture of cyclohexane and dichloromethane (2:1). To the solution were added 214 mg of benzyl 2,2,2-trichloroacetimidate ($Cl_3CC(=NH)OCH_2Ph$) and 0.015 ml of trifluoromethanesulfonic acid, and the mixture was reacted for 2 hours at room temperature. The reaction mixture was diluted with chloroform, and the dilution was washed successively with a saturated aqueous solution of sodium hydrogencarbonate and water in this order, and then concentrated. The residue was subjected to 30 ml silica gel column chromatography (developer system: toluene-ethyl acetate = 6:1) for separation and purification, to obtain 164 mg (83%) of the captioned compound as a syrup.
$[\alpha]_D^{26}$ - 101° (c 1.5, chloroform)

(6) Preparation of methyl 2,3-anhydro-4-0-benzyl-6-deoxy-α-L-gulopyranoside

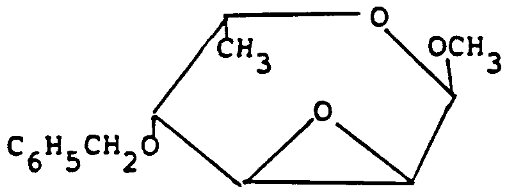

19.72 Grams of methyl 2-0-acetyl-4-0-benzyl-6-deoxy-3-0-tosyl-α-L-galactopyranoside was dissolved in 400 ml of anhydrous methanol, and 123 ml of a 28% sodium methoxide solution in methanol was added, followed by reacting the mixture for 4.5 hours at room temperature. Then carbon dioxide was introduced to the reaction mixture, followed by concentration. The residue was dissolved in 300 ml of chloroform, and the resulting solution was washed with water, and concentrated. The residue was purified by 800 ml silica gel column chromatography (developer system: hexane-acetone = 3:1) to obtain 6.62 g (62%) of the captioned compound as a colorless syrup.
$[\alpha]_D^{26}$ - 25° (c 3, chloroform)

(7) Preparation of methyl 4-0-benzyl-2,6-deoxy-2-fluoro-α-L-idopyranoside

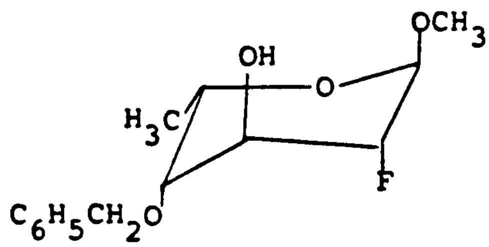

140 Milligrams of methyl 2,3-anhydro-4-0-benzyl-6-deoxy-α-L-gulopyranoside was dissolved in 2.8 ml of anhydrous ethylene glycol, and 880 mg of potassium hydrogenfluoride ($KHF_2$) was added, followed by stirring the mixture for 3 hours at 180°C. The reaction mixture was diluted with chloroform, and the dilution was washed successively with a saturated aqueous solution of sodium hydrogencarbonate and water in this order. This solution was concentrated, and the residue was purified by 30 ml silica gel column chromatography (developer system: hexane-acetone = 3:1), to obtain 67 mg (44 %) of the captioned compound as a syrup.
$[\alpha]_D^{26}$ - 62° (c 2, chloroform)

(8) Preparation of methyl 4-0-benzyl-2,6-dideoxy-2-fluoro-α-L-lyxo-hexopyranosid-3-ulose

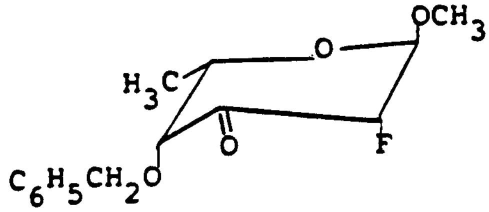

139 Milligrams of methyl 4-0-benzyl-2,6-dideoxy-2-fluoro-α-L-idopyranoside was dissolved in a mixture of 1 ml of anhydrous benzene and 0.14 ml of anhydrous dimethyl sulfoxide (acting as a solvent and an oxidizing agent). To the solution were added 155 mg of dicyclohexylcarbodiimide, 0.01 ml of anhydrous pyridine, and 23 mg of pyridinium trifluoroacetate, and the mixture was stirred for 3 hours at room

temperature. To the reaction mixture was added a methanol solution of 142 mg of oxalic acid, and the mixture was diluted with 30 ml of benzene. The insolubles were removed by filtration. The filtrate was washed successively with a saturated aqueous solution of sodium hydrogencarbonate and water in this order, and then concentrated. The residue was separated and purified by 25 ml silica gel column chromatography (developer system: hexane-acetone, 3:1), to obtain 110 mg (79%) of the captioned compound as needle crystals.
mp. 63 - 64°C
$[\alpha]_D^{26}$ - 7° (c 1, chloroform)

(9) Preparation of methyl 4-0-benzyl-2,6-dideoxy-2-fluoro-α-L-talopyranoside

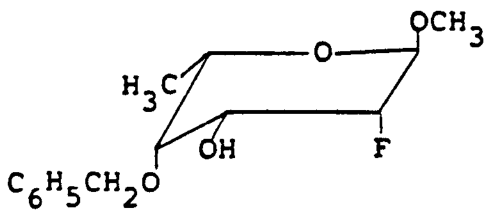

698 Milligrams of methyl 4-0-benzyl-2,6-dideoxy-2-fluoro-α-L-lyxo-hexapyranosid-3-ulose was dissolved in 14 ml of anhydrous tetrahydrofuran, and cooled to -30°C. To this solution was added a suspension of 198 mg of lithium aluminium hydride in 2 ml of anhydrous tetrahydrofuran. The mixture was stirred for 45 minutes at -30°C, 2 hours at -10°C, then 30 minutes at 0°C. The reaction mixture was cooled at 0°C, and a saturated aqueous solution of ammonium chloride was added. Then, 50 ml of chloroform was added, followed by filtering the mixture. The chloroform solution was washed with water, and concentrated. The residue was separated and purified by 100 ml silica gel column chromatography (developer system: hexane-acetone = 3:1) to obtain 576 mg (82%) of the captioned compound as a partially crystallized syrup.
$[\alpha]_D^{26}$ - 98° (c 3.7, chloroform)

(10) Preparation of methyl 2,6-dideoxy-2-fluoro-α-L-talopyranoside

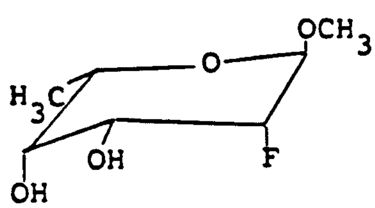

345 Milligrams of methyl 4-0-benzyl-2,6-dideoxy-2-fluoro-α-L-talopyranoside was dissolved in 8 ml of a mixture of dioxane, acetic acid and water (10:1:1). The solution was catalytically reduced at atmospheric pressure in the presence of palladium black to remove the benzyl group. The reaction mixture was filtered, and the filtrate was concentrated under reduced pressure to obtain 230 mg of a colorless solid.

To purify the colorless solid, recrystallization from chloroform-hexane was performed, whereby 186 mg (81%) of the captioned compound was obtained as colorless crystals.
mp. 112 - 114°C
$[\alpha]_D^{26}$ - 124° (c 1, methanol)

Referential Example 2

(1) Preparation of 1,3,4-tri-0-acetyl-2,6-dideoxy-2-fluoro-α-L-talopyranose

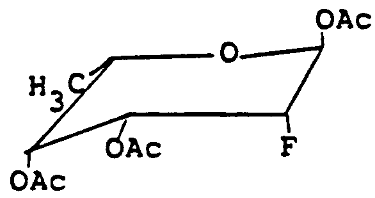

Ac = Acetyl group

230 Milligrams of the methyl 2,6-dideoxy-2-fluoro-α-L-talopyranoside obtained in Referential Example 1-(10), was dissolved in 7.6 ml of anhydrous nitromethane. To the solution were added 1.3 ml of acetic anhydride and 0.036 ml of sulfuric acid, and the mixture was reacted for 4 hours at room temperature. To neutralize the reaction mixture there was added a saturated aqueous solution of sodium hydrogencarbonate, and then the mixture was diluted with 50 ml of chloroform. The resulting dilution was washed with water and concentrated. The residue was separated and purified by 60 ml silica gel column chromatography (developer system: hexane-acetone = 3:1) to obtain 313 mg (84%) of the captioned compound as colorless crystals. Recrystallization was performed from ether-hexane.
mp. 102 - 103 ° C
$[\alpha]_D^{26}$ - 111 ° (c 1, chloroform)

(2) Preparation of 3,4-di-0-acetyl-2,6-dideoxy-2-fluoro-α-L-talpyranosyl bromide

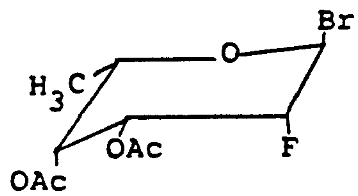

327 Milligams of 1,3,4-tri-0-acetyl-2,6-dideoxy-2-fluoro-α-L-talpyranose was dissolved in 7 ml of a mixture of anhydrous dichloromethane and anhydrous ethyl acetate (10:1), and 534 mg of titanium tetrabromide was added, followed by reacting the mixture for 22 hours at room temperature. To the reaction mixture were added 10 ml of anhydrous acetonitrile, 1.67 g of anhydrous sodium acetate, and 20 ml of anhydrous toluene in this order. The precipitate formed was removed by filtration, and the filtrate was concentrated under reduced pressure. To the residue was added 20 ml of anhydrous toluene, and the insolubles were removed by filtration. The filtrate was concentrated under reduced pressure to obtain 330 mg (94%) of the captioned compound as a syrup.
$^1$H-NMR spectrum (deuterochloroform):
δ 6.55 (1H, broad d, H-1)
4.81 (1H, ddt, H-2)

Referential Example 3

(1) Preparation of 7-0-(3,4-di-0-acetyl-2,6-dideoxy-2-fluoro-α-L-talopyranosyl)daunomycinone

A solution of 330 mg of 3,4-di-0-acetyl-2,6-dideoxy-2-fluoro-α-L-talopyranosyl bromide, obtained in Referential Example 2 (2), in 9 ml of anhydrous dichloromethane was added to a suspension of 290 mg of daunomycinone, 943 mg of yellow mercuric oxide, 273 mg of mercuric bromide, and 4.5 g of powdery molecular sieve 3A in 36 ml of anhydrous dichloromethane. The resulting mixture was stirred for 20 hours at room temperature in the dark. The reaction mixture was filtered, and the filtrate was diluted with chloroform. The dilution was washed successively with a 30% aqueous solution of potassium iodide, a saturated aqueous solution of sodium hydrogencarbonate, and water in this order, and then concentrated. The residue was purified and separated by 60 ml silica gel column chromatography (developer system:

benzene-acetone = 4:1) to obtain 378 mg (82%) of the captioned compound as a red solid. Reprecipitation was performed using chloroform-hexane.
mp. 144 - 146 °C
$[\alpha]_D^{26}$ + 211° (c 0.036, chloroform)

(2) Preparation of 7-0-(2,6-dideoxy-2-fluoro-α-L-talopyranosyl) daunomycinone

100 Milligrams of 7-0-(3,4-di-0-acetyl-2,6-dideoxy-2-fluoro-α-L-talopyranosyl)daunomycinone obtained in the above step (1) was dissolved in 8 ml of an aqueous solution of 0.2 N sodium hydroxide. The solution was reacted for 5 hours at 0 °C to remove the acetyl group by hydrolysis. At the same temperature, 1.6 ml of 1N hydrochloric acid was added to the reaction mixture for neutralization. Then, 1.5 g of sodium chloride was added, and the mixture was extracted with chloroform. The extract was washed with a saturated aqueous solution of sodium chloride, and concentrated. The resulting red solid was reprecipitated from chloroform-hexane to obtain the captioned compound as a red solid. The yield was 62 mg (72%).
$[\alpha]_D^{25}$ + 197° (c 0.02, chloroform-methanol (1:1))

Referential Example 4

Preparation of 7-0-(2,6-dideoxy-2-fluoro-3,4-0-isopropylidene-α-L-talopyranosyl)-14-bromo-daunomycinone

37.8 Milligrams of 7-0-(2,6-dideoxy-2-fluoro-α-L-talopyranosyl)daunomycinone obtained in Referential Example 3 was suspended in a mixture of 0.9 ml of anhydrous methanol and 1.4 ml of anhydrous dioxane. To the suspension was added 0.052 ml of methyl ortho-formate, and the mixture was subjected to react for the protection of the carbonyl group at the 13-position by ketal formation. The reaction mixture was then cooled to 0 °C, and a solution of 15 mg of bromine in 0.15 ml of anhydrous dichloromethane was added to the resulting suspension. The mixture was stirred for 1 hour at the same temperature, and then stirred for 1.5 hours at room temperature, thereby brominating the methyl group at the 14-position. The resulting homogeneous solution was added dropwise to 12 ml of isopropyl ether, and the red precipitate formed was collected by centrifugation, followed by washing the precipitate with isopropyl ether twice. Then, the precipitate was suspended in 3 ml of acetone, and the suspension was stirred for 40 minutes at room temperature. The removal of the ketal group took place while restoring the carbonyl group at the 13-position, and the introduction of an isopropylidene group with acetone also occurred. To the resulting homogenous solution were added 5 ml of isopropyl ether and 20 ml of hexane, and the precipitate formed was collected by centrifugation, to obtain 35 mg of 7-0-(2,6-dideoxy-2-fluoro-3,4-0-isopropylidene-α-L-talopyranosyl)-14-bromo-daunomycinone as a red solid.

**Claims**

**Claims for the following Contracting States : AT, BE, CH, DE, FR, GB, IT, LI, LU, NL, SE**

**1.** A compound of the general formula (I)

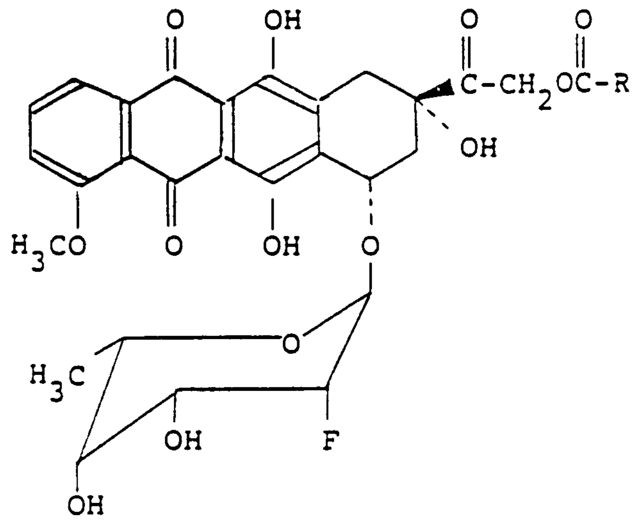

(I)

wherein R represents the group $-(CH_2)_mH$ in which m denotes 0 or an integer of 1 to 6, or the group $-(CH_2)_nCOOH$ in which n denotes 0 or an integer of 1 to 10.

2. A compound as defined in claim 1 wherein R is the group $-(CH_2)_nCOOH$ and n denotes an integer of 2 to 6.

3. A compound as claimed in Claim 1 which is 7-0-(2,6-dideoxy-2-fluoro-α-L-talopyranosyl)adriamycinone 14-0-hemipimelate.

4. A pharmaceutical composition comprising as the active ingredient a compound of the formula I as defined in claim 1 in association with a pharmaceutically acceptable carrier.

5. Use of a compound of the formula I as defined in claim 1 for the preparation of a medicament having anti-tumor activity.

6. A process for preparing an anthracycline derivative of the general formula I

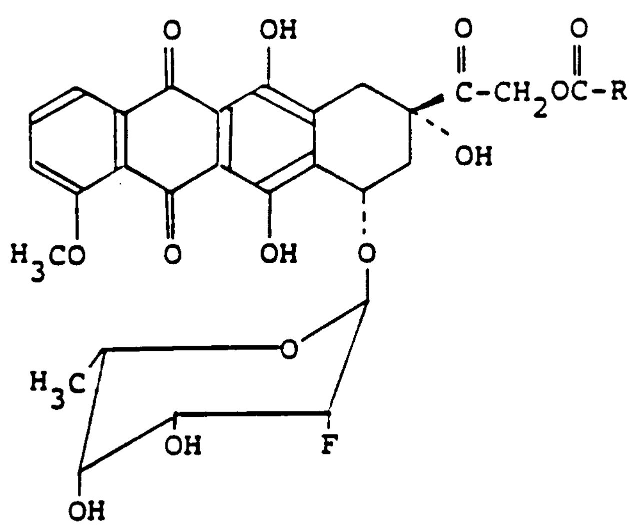

(I)

wherein R represents the group $-(CH_2)_mH$ in which m denotes 0 or an integer of 1 to 6, or the group $-(CH_2)_nCOOH$ in which n denotes 0 or an integer of 1 to 10, which comprises reacting a compound of

the general formula (II)

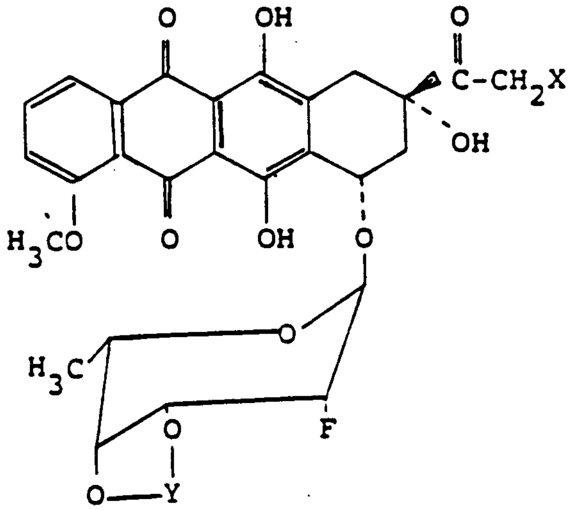

(II)

wherein X represents a bromine, chlorine or iodine atom, and Y represents a divalent hydroxyl-protecting group, or the corresponding compound from which the hydroxyl-protecting group Y is removed, with a compound of the formula (III)

A-OOC-R (III)

wherein R is as defined above, and A represents an alkali metal, to form a compound of the formula (IV)

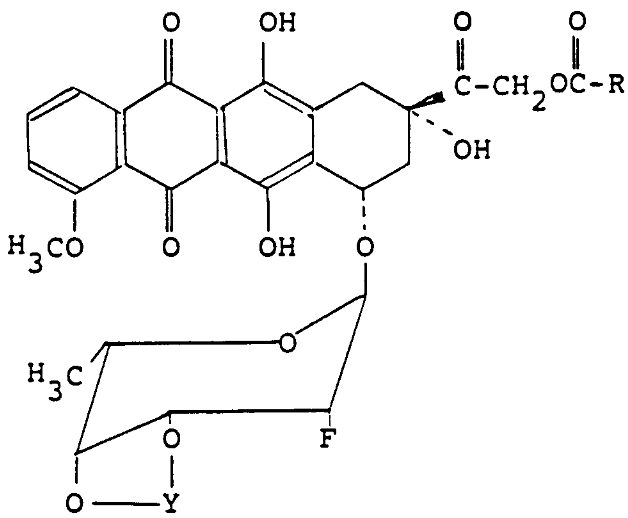

(IV)

wherein R and Y are as defined above, or the corresponding compound from which the hydroxyl-protecting group Y is removed; and then, if the resulting compound contains the hydroxyl-protecting group Y remaining therein, removing the hydroxyl-protecting group Y from said compound by a customary method.

**Claims for the following Contracting States : ES, GR**

**1.** A process for preparing an anthracycline derivative of the general formula (I)

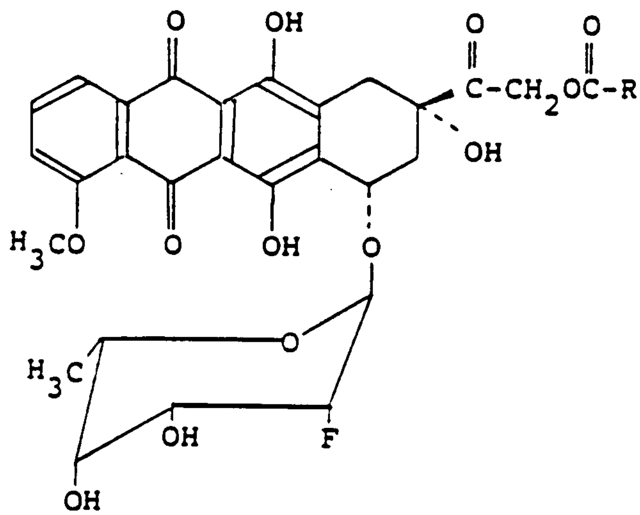

(I)

wherein R represents the group $-(CH_2)_mH$ in which m denotes 0 or an integer of 1 to 6, or the group $-(CH_2)_nCOOH$ in which n denotes 0 or an integer of 1 to 10, which comprises reacting a compound of the general formula (II)

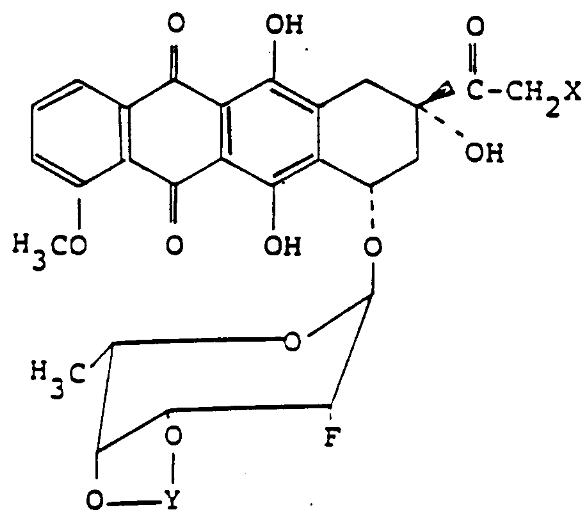

(II)

wherein X represents a bromine, chlorine or iodine atom, and Y represents a divalent hydroxyl-protecting group, or the corresponding compound from which the hydroxyl-protecting group Y is removed, with a compound of the formula (III)

A-OOC-R (III)

wherein R is as defined above, and A represents an alkali metal, to form a compound of the formula (IV)

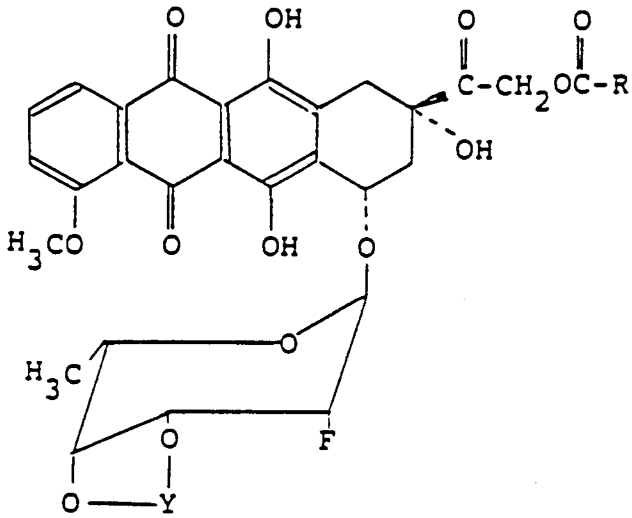

(IV)

wherein R and Y are as defined above, or the corresponding compound from which the hydroxyl-protecting group Y is removed; and then, if the resulting compound contains the hydroxyl-protecting group Y remaining therein, removing the hydroxyl-protecting group Y from said compound by a customary method.

2. A process as defined in claim 1, wherein R is the group $-(CH_2)_nCOOH$ and n denotes an integer of 2 to 6.

3. A process as defined in claim 1, wherein the compound obtained is 7-0-(2,6-dideoxy-2-fluoro-α-L-talopyranosyl)adriamycinone 14-0-hemipimelate.

4. Use of a compound of the formula I as defined in claim 1 for the preparation of a medicament having anti-tumor activity.

**Patentansprüche**
**Patentansprüche für folgende Vertragsstaaten : AT, BE, CH, DE, FR, GB, IT, LI, LU, NL, SE**

1. Verbindung der allgemeinen Formel (I),

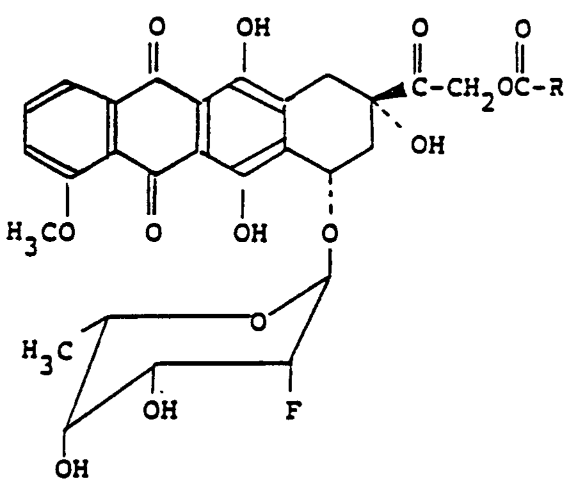

(I)

in der R eine Gruppe $-(CH_2)_mH$, in der m den Wert 0 oder eine ganze Zahl von 1 bis 6 bedeutet, oder eine Gruppe $-(CH_2)_nCOOH$ darstellt, in der n den Wert 0 oder eine ganze Zahl von 1 bis 10 bedeutet.

2. Verbindung gemäß Anspruch 1, in der R eine Gruppe $-(CH_2)_nCOOH$ darstellt, und n eine ganze Zahl von 2 bis 6 bedeutet.

3. Verbindung gemäß Anspruch 1, nämlich 7-0-(2,6-Dideoxy-2-fluor-α-L-talopyranosyl)adriamycinon-14-0-hemipimelat.

4. Arzneimittel umfassend als Wirkstoff eine Verbindung der Formel I gemäß Anspruch 1 in Verbindung mit einem pharmazeutisch verträglichen Träger.

5. Verwendung einer Verbindung der Formel I gemäß Anspruch 1 zur Herstellung eines Medikaments mit Antitumoraktivität.

6. Verfahren zur Herstellung eines Anthracyclinderivats der allgemeinen Formel (I),

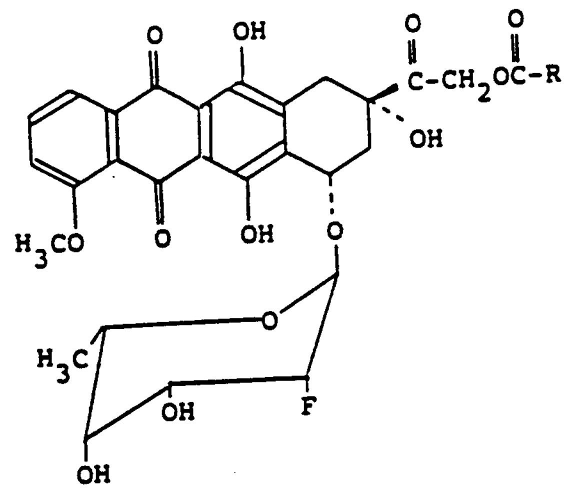

(I)

in der R eine Gruppe $-(CH_2)_mH$, in der m den Wert 0 oder eine ganze Zahl von 1 bis 6 bedeutet, oder eine Gruppe $-(CH_2)_nCOOH$ darstellt, in der n den Wert 0 oder eine ganze Zahl von 1 bis 10 bedeutet, umfassend die Reaktion einer Verbindung der allgemeinen Formel (II)

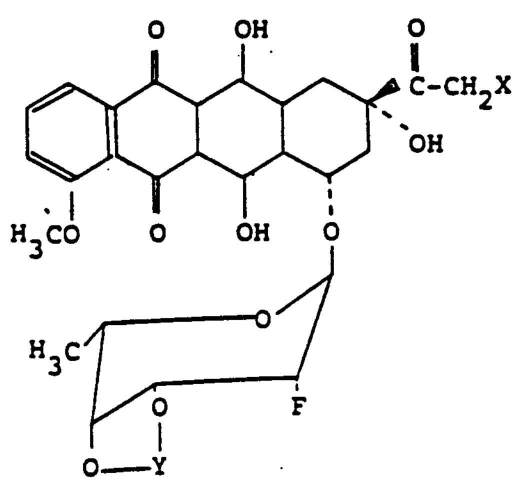

(II)

in der X ein Brom-, Chlor- oder Jodatom, und Y eine zweiwertige Hydroxylschutzgruppe ist, oder einer entsprechenden Verbindung, von der die Hydroxylschutzgruppe abgespalten wurde, mit einer Verbindung der Formel (III),

A-OOC-R (III)

in der R die vorstehend angegebene Bedeutung hat, und A ein Alkalimetall ist, wobei eine Verbindung der Formel (IV),

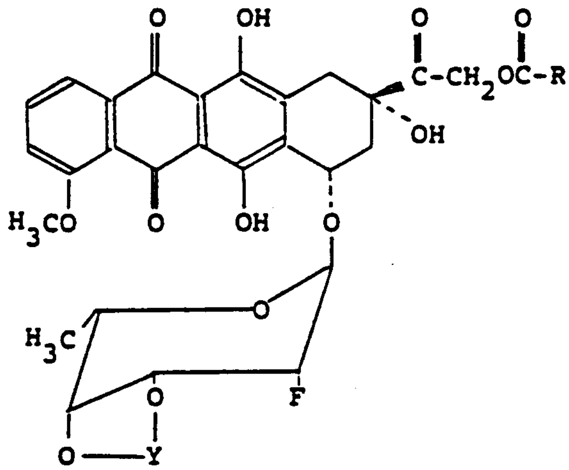

(IV)

in der R und Y die vorstehend angegebene Bedeutung haben, oder die entsprechende Verbindung, von der die Hydroxylschutzgruppe Y abgespalten wurde, erhalten wird; und danach, falls die resultierende Verbindung noch die Hydroxylschutzgruppe Y enthält, das Abspalten der Hydroxylschutzgruppe Y von besagter Verbindung nach einem üblichen Verfahren.

**Patentansprüche für folgende Vertragsstaaten : ES, GR**

**1.** Verfahren zur Herstellung eines Anthracyclinderivats der allgemeinen Formel (I),

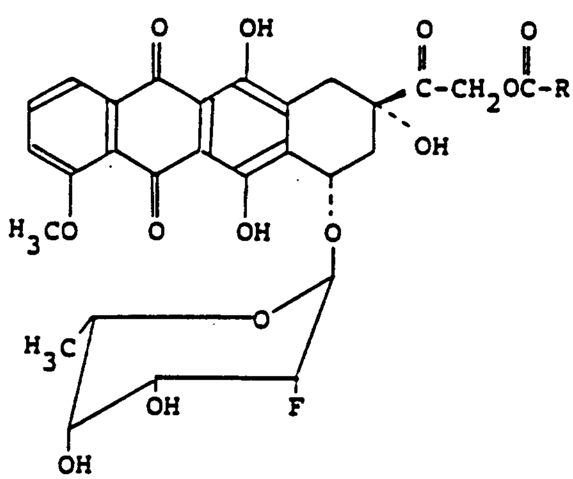

(I)

in der R eine Gruppe $-(CH_2)_mH$, in der m den Wert 0 oder eine ganze Zahl von 1 bis 6 bedeutet, oder eine Gruppe $-(CH_2)_nCOOH$ darstellt, in der n den Wert 0 oder eine ganze Zahl von 1 bis 10 bedeutet, umfassend die Reaktion einer Verbindung der allgemeinen Formel (II)

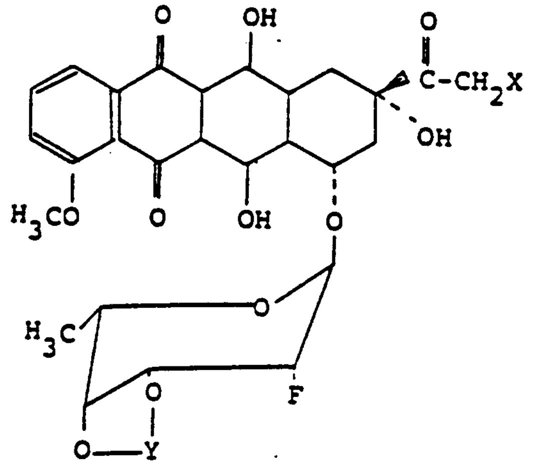

(II)

in der X ein Brom-, Chlor- oder Jodatom, und Y eine zweiwertige Hydroxylschutzgruppe ist, oder einer entsprechenden Verbindung, von der die Hydroxylschutzgruppe abgespalten wurde, mit einer Verbindung der Formel (III),

A-OOC-R (III)

in der R die vorstehend angegebene Bedeutung hat, und A ein Alkalimetall ist, wobei eine Verbindung der Formel (IV),

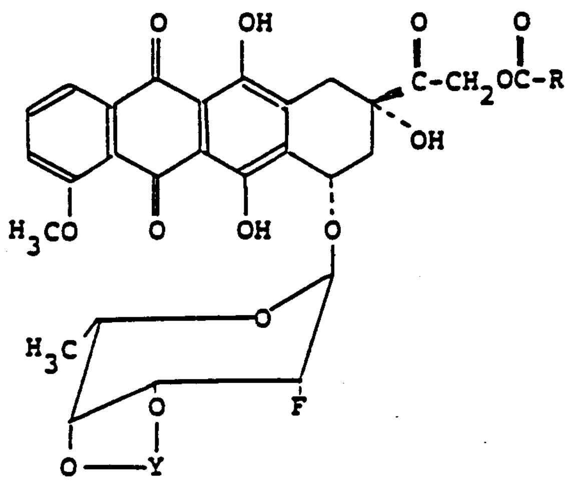

(IV)

in der R und Y die vorstehend angegebene Bedeutung haben, oder die entsprechende Verbindung, von der die Hydroxylschutzgruppe Y abgespalten wurde, erhalten wird; und danach, falls die resultierende Verbindung noch die Hydroxylschutzgruppe Y enthält, das Abspalten der Hydroxylschutzgruppe Y von besagter Verbindung nach einem üblichen Verfahren.

**2.** Verfahren gemäß Anspruch 1, in dem R eine Gruppe $-(CH_2)_nCOOH$ darstellt, und n eine ganze Zahl von 2 bis 6 bedeutet.

**3.** Verfahren gemäß Anspruch 1, in dem die erhaltene Verbindung 7-0-(2,6-Dideoxy-2-fluor-α-L-talopyranosyl)adriamycinon-14-0-hemipimelat ist.

**4.** Verwendung einer Verbindung der Formel I gemäß Anspruch 1 zur Herstellung eines Medikaments mit Antitumoraktivität.

**Revendications**

**Revendications pour les Etats contractants suivants : AT, BE, CH, DE, FR, GB, IT, LI, LU, NL, SE**

1. Composé de la formule générale (I) :

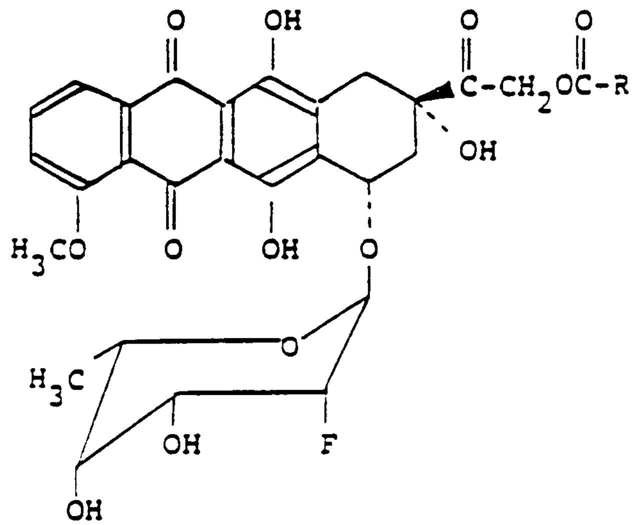

(I)

dans laquelle R représente le groupe $-(CH_2)_mH$ dans lequel m signifie 0 ou un nombre entier de 1 à 6, ou le groupe $-(CH_2)_nCOOH$ dans lequel n signifie 0 ou un nombre entier de 1 à 10.

2. Composé suivant la revendication 1, caractérisé en ce que R est le groupe $-(CH_2)_nCOOH$ et n signifie un nombre entier de 2 à 6.

3. Composé suivant la revendication 1, qui est constitué par le 14-0-hémipimélate de 7-0-(2,6-didésoxy-2-fluoro-α-L-talopyrannosyl)adriamycinone.

4. Composition pharmaceutique comprenant comme ingrédient actif un composé de la formule I tel que défini à la revendication 1, en association avec un support pharmaceutiquement acceptable.

5. Utilisation d'un composé de la formule I tel que défini à la revendication 1, pour la préparation d'un médicament ayant une activité antitumorale.

6. Procédé de préparation d'un dérivé d'anthracycline de la formule générale I :

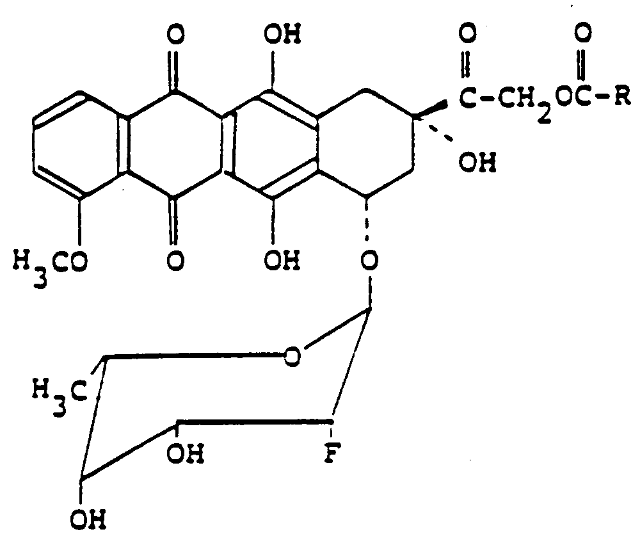

(I)

dans laquelle R représente le groupe $-(CH_2)_mH$ dans lequel m signifie 0 ou un nombre entier de 1 à 6, ou le groupe $-(CH_2)_nCOOH$ dans lequel n signifie 0 ou un nombre entier de 1 à 10, qui comprend la réaction d'un composé de la formule générale (II) :

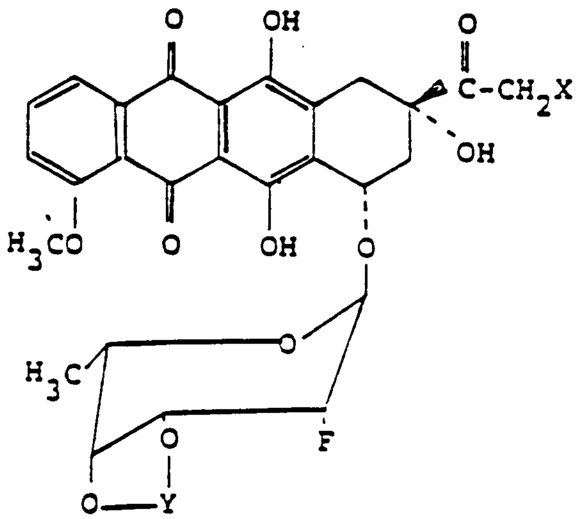

(II)

dans laquelle X représente un atome de brome, de chlore ou d'iode, et Y représente un groupe de protection d'hydroxyle bivalent, ou du composé correspondant dont le groupe de protection d'hydroxyle Y est séparé, avec un composé de la formule (III) :

A-OOC-R (III)

dans laquelle R est tel que défini précédemment, et A représente un métal alcalin, pour former un composé de la formule (IV) :

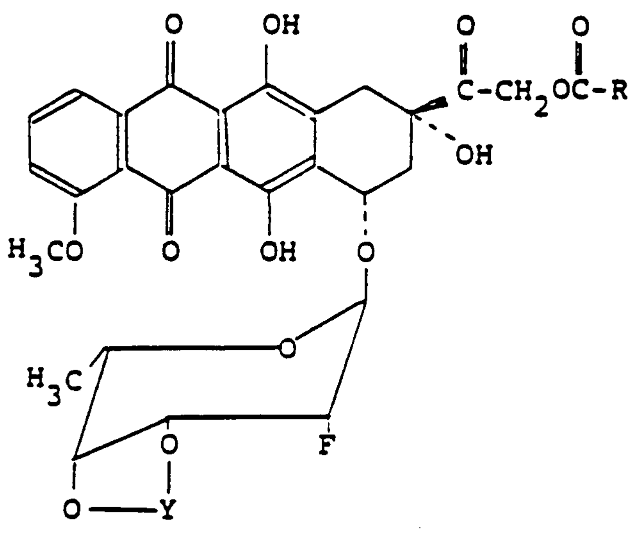

(IV)

dans laquelle R et Y sont tels que définis précédemment, ou le composé correspondant dont le groupe de protection d'hydroxyle Y est séparé, et ensuite, si le composé résultant contient le groupe de protection d'hydroxyle Y restant, la séparation du groupe de protection d'hydroxyle Y du composé précité par une méthode usuelle.

**Revendications pour les Etats contractants suivants : ES, GR**

**1.** Procédé de préparation d'un dérivé d'anthracycline de la formule générale (I) :

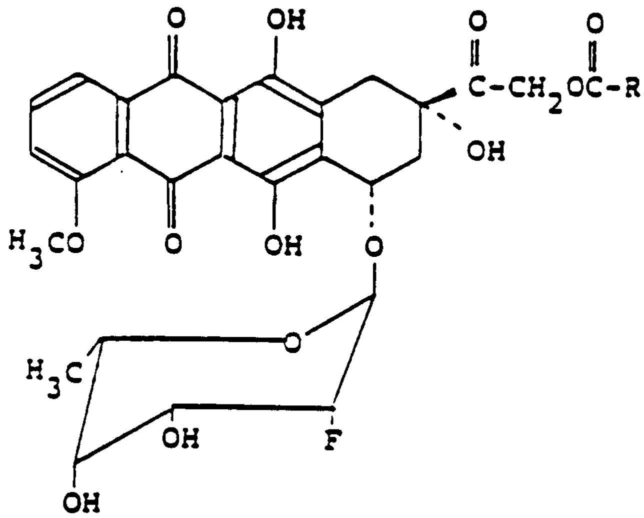

(I)

dans laquelle R représente le groupe $-(CH_2)_mH$ dans lequel m signifie 0 ou un nombre entier de 1 à 6, ou le groupe $-(CH_2)_nCOOH$ dans lequel n signifie 0 ou un nombre entier de 1 à 10, qui comprend la réaction d'un composé de la formule générale (II) :

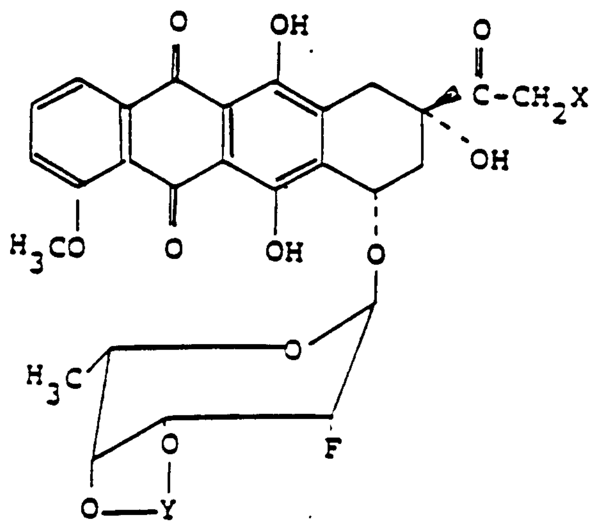

(II)

dans laquelle X représente un atome de brome, de chlore ou d'iode, et Y représente un groupe de protection d'hydroxyle bivalent, ou du composé correspondant dont le groupe de protection d'hydroxyle Y est séparé, avec un composé de la formule (III) :

A-OOC-R (III)

dans laquelle R est tel que défini précédemment, et A représente un métal alcalin, pour former un composé de la formule (IV) :

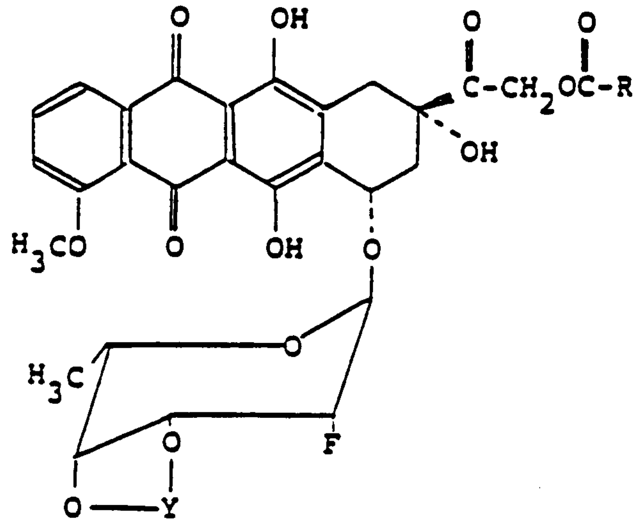

(IV)

dans laquelle R et Y sont tels que définis précédemment, ou le composé correspondant dont le groupe de protection d'hydroxyle est séparé, et ensuite, si le composé résultant contient le groupe de protection d'hydroxyle Y restant, la séparation du groupe de protection d'hydroxyle Y du composé précité par une méthode usuelle.

**2.** Procédé suivant la revendication 1, caractérisé en ce que R est le groupe $-(CH_2)_nCOOH$ et n signifie un nombre entier de 2 à 6.

**3.** Procédé suivant la revendication 1, caractérisé en ce que le composé obtenu est le 14-0-hémipimélate de 7-0-(2,6-didésoxy-2-fluoro-α-L-talopyrannosyl)adriamycinone.

**4.** Utilisation d'un composé de la formule I tel que défini à la revendication I, pour la préparation d'un médicament ayant une activité antitumorale.